# EUROPEAN PATENT SPECIFICATION

(11) **EP 3 990 036 B1**
(45) Date of publication and mention of the grant of the patent: **23.08.2023**
(21) Application number: 20743834.2
(22) Date of filing: 25.06.2020
(51) Int. Cl.: A61L 15/22, A61L 15/42, A61L 15/46

(54) **CITRIC ACID COATED WOUND DRESSING COMPOSITIONS AND METHODS OF MANUFACTURE**
ZITRONENSÄUREBESCHICHTETE WUNDVERBANDZUSAMMENSETZUNGEN UND HERSTELLUNGSVERFAHREN
COMPOSITIONS DE PANSEMENTS REVÊTUS D'ACIDE CITRIQUE ET LEURS PROCÉDÉS DE FABRICATION

(30) Priority: 28.06.2019 US 201962868611 P
(43) Date of publication of application: 04.05.2022
(73) Proprietor: KCI Licensing, Inc., San Antonio, TX 78265 (US)
(72) Inventor: LOCKE, Christopher Brian, San Antonio, Texas 78265 (US); DERRICK, Kathleen L., San Antonio, Texas 78265 (US); KIESWETTER, Kristine M., San Antonio, Texas 78265 (US)
(74) Representative: Simmons & Simmons
(86) International application number: PCT/IB2020/056042
(87) International publication number: WO 2020/261188

(56) References cited:
- WO-A1-2016/120621
- WO-A1-2019/040729

## Description

### TECHNICAL FIELD

The present technology relates generally to wound dressing compositions including a mixture of a collagen and/or chitosan and oxidized regenerated cellulose (ORC) and including a layer disposed within the composition and adjacent to an external surface that includes citric acid. Methods of manufacture as well as kits regarding the same are also provided.

### BACKGROUND

The following description of the background of the present technology is provided simply as an aid in understanding the present technology and is not admitted to describe or constitute prior art to the present technology.

Infections can retard wound healing and, if untreated, can result in tissue loss, systemic infections, septic shock, and death. Moreover, in addition to vegetative or free-floating bacteria present in a wound, bacterial biofilms may also form in a wound presenting further challenges in wound therapy, particularly chronic wounds. A biofilm is an association of microorganisms that can adhere to a surface forming three-dimensional microbial communities. The ability of bacteria to form these complex biofilms can impede a host's defense mechanisms against pathogens. Antimicrobials have been used to attempt to prevent, reduce, inhibit, and/or disrupt biofilm formation in wounds.

Document WO 2016/120621 A1 discloses a wound dressing comprising chitosan layer coated with a triprotic acid by dip coating followed by thermal drying. The triprotic acid is preferably citric acid and the layer can comprise additional components including collagen and/or oxidized regenerated cellulose.

### SUMMARY

In a first aspect of the present invention, a wound dressing composition as defined in claim 1 is provided.

In a second aspect according to the present invention, a method of manufacturing a wound dressing composition as defined in claim 13 is provided. In a third aspect of the present invention, a wound dressing composition as defined in claim 25 is provided.

In a further related aspect, a kit is provided that includes a wound dressing composition of any embodiment described herein as well as instructions for use.

In another related aspect, not claimed, a method for providing therapy to a tissue site that includes positioning a wound dressing composition of any embodiment of the present technology adjacent to the tissue site is provided. In any embodiment herein, the method for providing therapy may include providing negative pressure therapy to the tissue site.

In yet another related aspect, not claimed, a method of reducing or preventing the formation of a biofilm in a tissue site that includes applying a wound dressing composition of any embodiment of the present technology to the tissue site is provided.

The references to methods of treatment in the summary and detailed description of the invention in this description are to be interpreted as references to the compounds, pharmaceutical compositions and medicaments of the present invention for use in a method for treatment of the human (or animal) body by therapy.

### DETAILED DESCRIPTION

It is to be appreciated that certain aspects, modes, embodiments, variations and features of the present methods are described below in various levels of detail in order to provide a substantial understanding of the present technology.

### Definitions

The definitions of certain terms as used in this specification are provided below. Unless defined otherwise, all technical and scientific terms used herein generally have the same meaning as commonly understood by one of ordinary skill in the art to which this present technology belongs.

The following terms are used throughout as defined below.

As used herein and in the appended claims, singular articles such as "a", "an", and "the" and similar referents in the context of describing the elements (especially in the context of the following claims) are to be construed to cover both the singular and the plural, unless otherwise indicated herein or clearly contradicted by context. Recitation of ranges of values herein are merely intended to serve as a shorthand method of referring individually to each separate value falling within the range, unless otherwise indicated herein, and each separate value is incorporated into the specification as if it were individually recited herein. All methods described herein can be performed in any suitable order unless otherwise indicated herein or otherwise clearly contradicted by context. The use of any and all examples, or exemplary language (*e.g*., "such as") provided herein, is intended merely to better illuminate the embodiments and does not pose a limitation on the scope of the claims unless otherwise stated. No language in the specification should be construed as indicating any non-claimed element as essential.

As used herein, "about" will be understood by persons of ordinary skill in the art and will vary to some extent depending upon the context in which it is used. If there are uses of the term which are not clear to persons of ordinary skill in the art, given the context in which it is used, "about" will mean up to plus or minus 10% of the particular term - for example, "about 10 wt.%" would mean "9 wt.% to 11 wt.%." It is to be understood that when "about" precedes a term, the term is to be construed as disclosing "about" the term as well as the term without modification by "about" - for example, "about 10 wt.%" discloses "9 wt.% to 11 wt.%" as well as discloses "10 wt.%."

As understood by one of ordinary skill in the art, "molecular weight" (also known as "relative molar mass") is a dimensionless quantity but is converted to molar mass by multiplying by 1 gram/mole - for example, collagen with a weight-average molecular weight of 5,000 has a weight-average molar mass of 5,000 g/mol.

The term "substantially free" as used herein means the indicated component is present in an amount less than about 0.001 wt.% weight percent (wt.%) of the composition.

As used herein, the term "biofilm" refers to an association of microorganisms, *e.g*., single or multiple species, that can be encased or embedded in a matrix material, which may be self-produced by resident microorganisms. The biofilm may be present or adhere to living and/or non-living surfaces, *e.g*., tissue, a wound, medical implants, such as but not limited to orthopedic implants, dental implants, catheters, stents and so on. Exemplary microorganisms include, but are not limited to bacteria, *e.g.,* Gram-negative bacteria, such as *Pseudomonas aeruginosa,* Gram-positive bacteria, such as *Staphylococcus aureus* and *Streptococcus mutans,* and fungi, such as yeasts, *e.g., Candida albicans.* The term "matrix material" is intended to encompass extracellular polymeric substances. Exemplary matrix materials include, but are not limited to polysaccharides, glycoproteins and/or nucleic acids. The term "biofilm" is further intended to include biological films that develop and persist at interfaces in aqueous environments. The language "biofilm development" or "biofilm formation" is intended to include the formation, growth, and modification of the bacterial colonies contained with biofilm structures, as well as the synthesis and maintenance of the exopolysaccharide of the biofilm structures. "Reducing" or "disrupting" a biofilm includes reducing the number of total viable microorganisms making up at least part of the biofilm, for example, as measured by total viable counts (TVC) of microorganisms (*e.g.*, bacteria, yeast).

As used herein, the "administration" of a wound dressing composition to a subject includes any route of introducing or delivering to a subject a diagnostic wound dressing composition to perform its intended function. Administration can be carried out by any suitable route, including but not limited to, topical administration. Administration includes self-administration and the administration by another.

As used herein, the terms "individual", "patient", or "subject" can be an individual organism, a vertebrate, a mammal, or a human. In some embodiments, the individual, patient, or subject is a human.

"Treating" or "treatment" as used herein covers the treatment of a wound described herein, in a subject, such as a human, and includes: (i) inhibiting a wound, *i.e.,* arresting its development; (ii) relieving a wound, *i.e.,* causing regression of the wound; (iii) slowing progression of the wound; and/or (iv) inhibiting, relieving, or slowing progression of one or more symptoms of the wound. In some embodiments, treatment means that the symptoms associated with the wound are alleviated, reduced, cured, or placed in a state of remission.

"Tissue site" as used herein refers to a wound, defect, or other treatment target located on or within tissue, including but not limited to, bone tissue, adipose tissue, muscle tissue, neural tissue, dermal tissue, vascular tissue, connective tissue, cartilage, tendons, or ligaments. A wound may include chronic, acute, traumatic, subacute, and dehisced wounds, partial-thickness burns, ulcers (such as diabetic, pressure, or venous insufficiency ulcers), flaps, grafts, or a combination of any two or more thereof. The term "tissue site" may also refer to areas of any tissue that are not necessarily wounded or defective, but are instead areas in which it may be desirable to add or promote the growth of additional tissue.

It is also to be appreciated that the various modes of treatment of wounds as described herein are intended to mean "substantial," which includes total but also less than total treatment, and wherein some biologically or medically relevant result is achieved. The treatment may be a continuous prolonged treatment for a chronic wound or a single administration, or a few administrations for the treatment of an acute wound.

### Wound Dressing Compositions of the Present Technology

A wound can become infected by microbes. An infected wound is a wound in which bacteria or other microorganisms have colonized, causing a deterioration and delay in the healing of the wound. Thus, a reduction in bacterial colonization is vital in wound therapy.

A biofilm is an association of microorganisms (*e.g*., single or multiple species) that can adhere to a surface forming three-dimensional microbial communities, which can have coordinated multi-cellular behavior. Typically, biofilms can produce extracellular polysaccharides thereby forming an extracellular matrix in which the bacteria are embedded. The ability of bacteria to form these complex biofilms can impede a host's defense mechanisms against pathogens. As such, biofilms often display a heightened tolerance to antimicrobial treatment.

The present disclosure is directed to wound dressing compositions that include at least one layer of citric acid disposed within the wound dressing composition, which may prevent, reduce, inhibit, or disrupt biofilm levels in a wound upon application, and over time.

Thus, in an aspect, a wound dressing composition is provided that includes a first external surface and a second external surface. The wound dressing composition includes about 30 wt.% to about 95 wt.% of one or more of collagen and chitosan, and about 30 wt.% to about 70 wt.% oxidized regenerated cellulose (ORC). In any embodiment herein, the wound dressing composition may include a weight ratio of the one or more of collagen and chitosan to ORC from about 70:30 to about 30:70. The weight ratio of the one or more of collagen and chitosan to ORC may be about 70:30, about 69:31, about 68:32, about 67:33, about 66:34, about 65:35, about 64:36, about 63:37, about 62:38, about 61:39, about 60:40, about 59:41, about 58:42, about 57:43, about 56:44, about 55:45, about 54:46, about 53:47, about 52:48, about 51:49, about 50:50, about 49:51, about 48:52, about 47:53, about 46:54, about 45:55, about 44:56, about 43:57, about 42:58, about 41:59, about 40:60, about 39:61, about 38:62, about 37:63, about 36:64, about 35:65, about 34:66, about 32:68, about 31:69, about 30:70, or any range including and/or in between any two of these values.

A first layer that includes citric acid is disposed within the wound dressing composition adjacent to the first external surface of the wound dressing composition. In any embodiment disclosed herein, the first layer that includes citric acid may extend inward towards the interior of the wound dressing composition. In any embodiment disclosed herein, the wound dressing composition may also include a second layer disposed within the wound dressing composition adjacent to the second external surface, wherein the second layer independently includes citric acid. The first layer and (when present) second layer may each independently have an average thickness of about 10 µm to about 3,500 µm; thus, the first layer and (when present) second layer may each independently have an average thickness of about 10 µm, about 20 µm, about 30 µm, about 40 µm, about 50 µm, about 60 µm, about 70 µm, about 80 µm, about 90 µm, about 100 µm, about 150 µm, about 200 µm, about 250 µm, about 300 µm, about 350 µm, about 400 µm, about 450 µm, about 500 µm, about 550 µm, about 600 µm, about 650 µm, about 700 µm, about 750 µm, about 800 µm, about 850 µm, about 900 µm, about 950 µm, about 1,000 µm, about 1,250 µm, about 1,500 µm, about 1,750 µm, about 2,000 µm, about 2,250 µm, about 2,500 µm, about 2,750 µm, about 3,000 µm, about 3,250 µm, about 3,500 µm, or any range including an/or in between any two of these values. The average thickness may be determined by using a calibrated optical microscope to measure the citric acid-containing band of a cross-section of the wound dressing composition. While a variety of methods of determining the average thickness of a layer may be utilized, one approach includes measuring the layer thickness of ten (10) equidistant points of a cross-section of the wound dressing composition and calculating the average from the ten measurements. Citric acid may be included in the first layer and (when present) the second layer at the same or differing amounts. The amount of citric acid included in the first layer and (when present) the second layer may independently for each layer be about 0.001 wt.% to about 20 wt.% based on the total weight of the wound dressing composition; thus, the amount of citric acid included in the first layer and (when present) the second layer may independently for each layer be (by total weight of the wound dressing composition) about 0.001 wt.%, about 0.002 wt.%, about 0.003 wt.%, about 0.004 wt.%, about 0.005 wt.%, about 0.006 wt.%, about 0.007 wt.%, about 0.008 wt.%, about 0.009 wt.%, about 0.01 wt.%, about 0.02 wt.%, about 0.03 wt.%, about 0.04 wt.%, about 0.05 wt.%, about 0.06 wt.%, about 0.07 wt.%, about 0.08 wt.%, about 0.09 wt.%, about 0.1 wt.%, about 0.2 wt.%, about 0.3 wt.%, about 0.4 wt.%, about 0.5 wt.%, about 0.6 wt.%, about 0.7 wt.%, about 0.8 wt.%, about 0.9 wt.%, about 1 wt.%, about 2 wt.%, about 3 wt.%, about 4 wt.%, about 5 wt.%, about 0.6 wt.%, about 0.7 wt.%, about 0.8 wt.%, about 0.9 wt.%, about 1 wt.%, about 2 wt.%, about 3 wt.%, about 4 wt.%, about 5 wt.%, about 6 wt.%, about 7 wt.%, about 8 wt.%, about 9 wt.%, about 10 wt.%, about 11 wt.%, about 12 wt.%, about 13 wt.%, about 14 wt.%, about 15 wt.%, about 16 wt.%, about 17 wt.%, about 18 wt.%, about 19 wt.%, about 20 wt.%, or any range including and/or in between any two of these values.

While the first layer and (when present) the second layer each include citric acid, the remaining wound dressing composition may be substantially free of citric acid. In any embodiment herein, the amount of citric acid in the remaining wound dressing composition (*i.e.,* not including the first layer and (when present) the second layer) may be less than about 0.0001 wt.%. However, in any embodiment herein, it may be that the first layer extends the entire thickness of the wound dressing composition (where there would be no second layer) and there is a gradient of concentration of the citric acid between the first external surface and the second external surface. The wound dressing composition may have an average thickness of about 0.01 cm to about 0.5 cm; thus, the thickness of the wound dressing composition may be about 0.01 cm, about 0.02 cm, about 0.03 cm, about 0.04 cm, about 0.05 cm, about 0.06 cm, about 0.07 cm, about 0.08 cm, about 0.09 cm, about 0.10 cm, about 0.15 cm, about 0.20 cm, about 0.25 cm, about 0.30 cm, about 0.35 cm, about 0.40 cm, about 0.45 cm, about 0.50 cm, or any range including and/or in between any two of these values

The first layer and (when present) the second layer may each independently include a dye marker. The dye marker of the first and/or second layer may independently for each layer be E124, fluorescein isothiocyanate (FITC), 4',6-diamidino-2-phenylindole (DAPI), methylene blue, erythrosine B, ponceau S, alura red, SYBR green, alcian blue, brilliant blue G, calcein blue, cardio green, crystal violet, nile blue, india ink, brilliant blue, indigo carmine, sudan III, methyl green, oil red, pyronin Y, tattoo ink, purpurin, phloxine B, picric acid, carbon nanotubes, a fuchsin, resazurin, a trichrome, or a combination of any two or more thereof. For example, the first and/or second layer may independently include a mixture of FITC, erythrosine B, ponceau S, brilliant blue and cardio green. As another example, the first and/or second layer may independently include a mixture of methylene blue, SYBR green, and purpurin. Yet another example is where the first and/or second layer independently include a mixture of oil red, nile blue and sudan III. In any embodiment herein, it may be that the first external surface is a wound-facing external surface. In any embodiment herein, it may be the second external surface is an environmental-facing external surface.

In any embodiment disclosed herein, the collagen of the wound dressing composition may include a mammalian collagen, such as a bovine collagen, a human collagen, or a combination thereof. The bovine collagen may include one or both of bovine collagen type I and bovine collagen type III. Exemplary types of human collagen include, but are not limited to, human collagen type I, human collagen type II, human collagen type III, human collagen type IV, human collagen type V, human collagen type VI, human collagen type VII, human collagen type VIII, human collagen type IX, human collagen type X, human collagen type XI, human collagen type XII, human collagen type XIII, human collagen type XIV, human collagen type XV, human collagen type XVI, human collagen type XVII, human collagen type XVIII, human collagen type XIX, human collagen type XX, human collagen type XXI, human collagen type XXII, human collagen type XXIII, human collagen type XXIV, human collagen type XXV, human collagen type XXVI, human collagen type XXVII, as well as a combination of any two or more thereof. The collagen of any embodiment herein may be a Type I collagen, a Type II collagen, a Type III collagen, may be obtained from any natural source, may be chemically-modified collagen (*e.g.,* an atelocollagen obtained by removing the immunogenic telopeptides from natural collagen), or may be a combination of any two or more thereof. For example, the collagen may include collagen obtained from bovine corium that has been rendered largely free of non-collagenous components, for example, including fat, non-collagenous proteins, polysaccharides, and other carbohydrates, such as by procedures described in U.S. Pat. Nos. 4,614,794 and 4,320,201. In any embodiment disclosed herein, the wound dressing composition may include a weight ratio of human collagen type I to human collagen type III of about 100:0, about 90:10, about 80:20, about 70:30, about 60:40, about 50:50, about 40:60, about 30:70, about 20:80, about 10:90, about 0:100, or any range including and/or in between any two of these values. The ratio by weight of human collagen type I to human collagen type III may be greater than about 50:50, or greater than about 70:30. The collagen of any embodiment herein may include a weight ratio of type I bovine collagen to type III bovine collagen of about 85:15.

As discussed above, the wound dressing composition includes about 30 wt.% to about 95 wt.% of one or more of collagen and chitosan. Thus, the amount of collagen and/or chitosan in the wound dressing composition may be about 30 wt.%, about 32 wt.%, about 34 wt.%, about 36 wt.%, about 38 wt.%, about 40 wt.%, about 42 wt.%, about 44 wt.%, about 46 wt.%, about 48 wt.%, about 50 wt.%, about 52 wt.%, about 54 wt.%, about 56 wt.%, about 58 wt.%, about 60 wt.%, about 62 wt.%, about 64 wt.%, about 66 wt.%, about 68 wt.%, about 70 wt.%, about 72 wt.%, about 74 wt.%, about 76 wt.%, about 78 wt.%, about 80 wt.%, about 82 wt.%, about 84 wt.%, about 86 wt.%, about 88 wt.%, about 90 wt.%, about 92 wt.%, about 94 wt.%, about 95 wt.%, or any range including and/or in between any two of these values. The collagen included in the wound dressing composition may have a weight-average molecular weight of about 5,000 to about 100,000. For example, in any embodiment disclosed herein, the collagen may have a weight-average molecular weight of about 5,000, about 6,000, about 7,000, about 8,000, about 9,000, about 10,000, about 12,000, about 14,000, about 16,000, about 18,000, about 20,000, about 22,000, about 24,000, about 26,000, about 28,000, about 30,000, about 32,000, about 34,000, about 36,000, about 38,000, about 40,000, about 45,000, about 50,000, about 55,000, about 60,000, about 65,000, about 70,000, about 75,000, about 80,000, about 85,000, about 90,000, about 95,000, about 100,000, or any range including and/or in between any two of these values. The chitosan of the wound dressing composition may have a weight-average molecular weight of about 100 to about 220,000; thus, in any embodiment herein, the chitosan of the wound dressing may have a weight-average molecular weight of about 100, about 200, about 300, about 400, about 500, about 600, about 700, about 800, about 900, about 1,000, about 2,000, about 3,000, about 4,000, about 5,000, about 6,000, about 7,000, about 8,000, about 9,000, about 10,000, about 12,000, about 14,000, about 16,000, about 18,000, about 20,000, about 22,000, about 24,000, about 26,000, about 28,000, about 30,000, about 32,000, about 34,000, about 36,000, about 38,000, about 40,000, about 45,000, about 50,000, about 55,000, about 60,000, about 65,000, about 70,000, about 75,000, about 80,000, about 85,000, about 90,000, about 95,000, about 100,000, about 120,000, about 140,000, about 160,000, about 180,000, about 200,000, about 220,000, or any range including and/or in between any two of these values.

Oxidized regenerated cellulose (ORC) may be produced by the oxidation of cellulose, for example with dinitrogen tetroxide and/or as described in U.S. Pat. No. 3,122,479. Not intending to be bound by theory, it is believed that this process may convert primary alcohol groups on the saccharide residues of the cellulose to carboxylic acid groups, for example, forming uronic acid residues within the cellulose chain. The oxidation may not proceed with complete selectivity, and as a result hydroxyl groups on carbons 2 and 3 of the saccharide residue may be converted to the keto form. These ketone units may introduce an alkali labile link, which at pH 7 or higher initiates the decomposition of the polymer *via* formation of a lactone and sugar ring cleavage. As a result, oxidized regenerated cellulose is biodegradable and bioresorbable under physiological conditions. ORC is available with a variety of degrees of oxidation and hence rates of degradation. The ORC may include particles, fibers, or both; in any embodiment disclosed herein, the ORC may be in the form of particles, such as fiber particles or powder particles. In embodiments that include ORC fibers, the ORC fibers may have a volume fraction such that at least 80% of the fibers have lengths in the range from about 5 µm to about 1000 µm, or from about 250 µm to about 450 µm.

As discussed above, the ORC of the wound dressing composition may include about 30 wt.% to about 70 wt.% based on the weight of the wound dressing composition of the ORC. In any embodiment disclosed herein, the ORC may have a weight-average molecular weight of about 50,000 to about 1,000,000. Thus, the wound dressing composition may include an ORC of any embodiment disclosed herein in an amount of about 30 wt.%, about 32 wt.%, about 34 wt.%, about 36 wt.%, about 38 wt.%, about 40 wt.%, about 42 wt.%, about 44 wt.%, about 46 wt.%, about 48 wt.%, about 50 wt.%, about 52 wt.%, about 54 wt.%, about 56 wt.%, about 58 wt.%, about 60 wt.%, about 62 wt.%, about 64 wt.%, about 66 wt.%, about 68 wt.%, about 70 wt.%, or any range including and/or in between any two of these values based on the weight of the wound dressing composition. The ORC may have a weight-average molecular weight of about 50,000, about 100,000, about 150,000, about 200,000, about 250,000, about 300,000, about 350,000, about 400,000, about 450,000, about 500,000, about 550,000, about 600,000, about 650,000, about 700,000, about 750,000, about 800,000, about 850,000, about 900,000, about 950,000, about 1,000,000, or any range including and/or in between any two of these values.

In any embodiment disclosed herein, the wound dressing composition may include about 0.001 wt.% to about 5 wt.% of an antimicrobial agent. Thus, the total amount of antimicrobial agent(s) in the wound dressing composition may be about 0.001 wt.%, about 0.002 wt.%, about 0.003 wt.%, about 0.004 wt.%, about 0.005 wt.%, about 0.006 wt.%, about 0.007 wt.%, about 0.008 wt.%, about 0.009 wt.%, about 0.01 wt.%, about 0.02 wt.%, about 0.03 wt.%, about 0.04 wt.%, about 0.05 wt.%, about 0.06 wt.%, about 0.07 wt.%, about 0.08 wt.%, about 0.09 wt.%, about 0.1 wt.%, about 0.2 wt.%, about 0.3 wt.%, about 0.4 wt.%, about 0.5 wt.%, about 0.6 wt.%, about 0.7 wt.%, about 0.8 wt.%, about 0.9 wt.%, about 1 wt.%, about 2 wt.%, about 3 wt.%, about 4 wt.%, about 5 wt.%, or any range including and/or in between any two of these values. The antimicrobial agent may include a penicillin, a streptomycin, ionic silver, chlorhexidine, a poly(hexamethylene biguanide) (PHMB), iodine, or a combination of any two or more thereof. Ionic silver may be provided in a variety of forms including as pharmaceutically acceptable salts, where representative examples include but are not limited to silver oxide, silver oxysalts, silver chromate, silver allantoinate, silver borate, silver glycerolate, silver nitrate, silver acetate, silver chloride, silver sulfate, silver lactate, silver bromide, silver iodide, silver carbonate, silver citrate, silver laurate, silver deoxycholate, silver salicylate, silver *p*-aminobenzoate, silver *p*-aminosalicylate, or a combination of any two or more thereof. In addition, while ionic silver may initially be incorporated into the wound dressing composition as a silver salt, at least a portion (if not substantially all) of the ionic silver may be complexed to the ORC in the wound dressing composition.

In any embodiment disclosed herein, the wound dressing composition may include one or more additional biomaterials such as gelatin, fibronectin, hyaluronic acid, polysaccharides, and a combination of any two or more thereof. The total amount of the one or more additional biomaterials in the wound dressing composition may be about 1 wt.%, about 2 wt.%, about 3 wt.%, about 4 wt.%, about 5 wt.%, about 6 wt.%, about 7 wt.%, about 8 wt.%, about 9 wt.%, about 10 wt.%, about 11 wt.%, about 12 wt.%, about 13 wt.%, about 14 wt.%, about 15 wt.%, about 16 wt.%, about 17 wt.%, about 18 wt.%, about 19 wt.%, about 20 wt.%, about 21 wt.%, about 22 wt.%, about 23 wt.%, about 24 wt.%, about 25 wt.%, or any range including and/or in between any two of these values, based on the weight of the wound dressing composition.

In any embodiment disclosed herein, in addition to citric acid the wound dressing composition may include about 0.001 wt.% to about 5 wt.% of one or more antioxidants. Thus, the total amount of antioxidants may be about 0.001 wt.%, about 0.002 wt.%, about 0.003 wt.%, about 0.004 wt.%, about 0.005 wt.%, about 0.006 wt.%, about 0.007 wt.%, about 0.008 wt.%, about 0.009 wt.%, about 0.01 wt.%, about 0.02 wt.%, about 0.03 wt.%, about 0.04 wt.%, about 0.05 wt.%, about 0.06 wt.%, about 0.07 wt.%, about 0.08 wt.%, about 0.09 wt.%, about 0.1 wt.%, about 0.2 wt.%, about 0.3 wt.%, about 0.4 wt.%, about 0.5 wt.%, about 0.6 wt.%, about 0.7 wt.%, about 0.8 wt.%, about 0.9 wt.%, about 1 wt.%, about 2 wt.%, about 3 wt.%, about 4 wt.%, about 5 wt.%, or any range including and/or in between any two of these values. Exemplary antioxidants include, but are not limited to, anthocyanins, astaxanthin, bilirubin, canthaxanthin, capsaicin, curcumin, coenzyme Q10, eugenol, flavanol, flavonolignans, flavanone, flavone, flavonol, iodide, isoflavone phytoestrogen, lutein, lycopene, manganese, melatonin, N-acetylcysteine, oxalic acid, phenolic acid, phytic acid, *R*-α-lipoic acid, stilbenoid, tocopherol, tocotrienol, vitamin A, vitamin C, vitamin E, xanthones, zeaxanthin, α-carotene, β-carotene, as well as combinations of any two or more thereof.

Examples of anthocyanins include, but are not limited to cyanidin, delphinidin, malvidin, pelargonidin, peonidin, petunidin, as well as combinations of any two or more combination thereof.

Examples of flavanols include, but are not limited to catechin, epicatechin, theaflavin, thearubigins, gallocatechin, epigallocatechin, or any gallate ester thereof, as well as combinations of any two or more thereof.

Examples of flavanones include, but are not limited to eriodictyol, hesperetin, naringenin, and combinations of any two or more thereof.

Examples of flavones include, but are not limited to apigenin, luteolin, tangeritin, and combinations of any two or more thereof.

Examples of flavonols include, but are not limited to isorhamnetin, kaempferol, myricetin, proanthocyanidins, quercetin, rutin, and combinations of any two or more thereof.

Examples of isoflavone phytoestrogens include, but are not limited to daidzein, genistein, glycitein, and combinations of any two or more thereof.

Examples of phenolic acids include, but are not limited to chicoric acid, chlorogenic acid, cinnamic acid, ellagic acid, ellagitannins, gallic acid, gallotannins, rosmarinic acid, salicylic acid, or any ester thereof, and combinations of any two or more thereof.

Examples of stillbenoids includes, but is not limited to resveratrol, pterostilbene, and mixtures thereof.

In any embodiment disclosed herein, the first layer and (when present) the second layer that includes citric acid may further include one or more binders.

In any embodiment disclosed herein, the wound dressing composition of the present technology may be capable of preventing, reducing, inhibiting, or disrupting biofilm formation in a wound. Reducing a biofilm includes reducing the number of total viable microorganisms making up at least part of the biofilm, for example, as measured by total viable counts (TVC) of microorganisms (*e.g.,* bacteria, yeast). The biofilm may include bacteria such as *Pseudomonas aeruginosa, Staphylococcus aureus* and *Streptococcus mutans.* The biofilm may also include fungi including, but not limited to, yeasts (such as *Candida albicans*)*.* The wound dressing composition of the present technology may be capable of preventing, reducing, inhibiting, and/or disrupting a biofilm in a wound by ≥ about 10% to about 100%, after 24 hours *in vitro* or *in vivo* exposure. For example, the wound dressing composition of the present technology may be capable of preventing, reducing, inhibiting, and/or disrupting a biofilm in a wound by about 10%, about 15%, about 20%, about 25%, about 30%, about 35%, about 40%, about 45%, about 50%, about 55%, about 60%, about 65%, about 70%, about 75%, about 80%, about 85%, about 90%, about 95%, about 99%, about 100%, or any range including and/or in between any two of these values.

The therapeutic efficacy of the wound dressing composition of the present technology can be assayed using any method known to those in the art. An exemplary method to test the therapeutic efficacy of the wound dressing composition of the present technology is the colony drip flow reactor (C-DFR) assay (*see* Lipp, C., et al., J. Wound Care, 19:220-226(2010).

In any embodiment disclosed herein, the wound dressing composition of the present disclosure may be sterile. In any embodiment disclosed herein, the wound dressing composition of the present disclosure may be packaged in a microorganism-impermeable container.

### Methods of Manufacturing Wound Dressing Compositions of the Present Technology

In a related aspect, a method of manufacturing a wound dressing composition of any embodiment of the present technology from a dressing composition that includes a first exterior surface and a second exterior surface is provided, where the method includes applying a first citric acid solution to the first exterior surface of the dressing composition. The first citric acidic solution includes a first solvent and about 50 mM to about 400 mM citric acid. The first citric acid solution may be applied at a volume (determined at 20 °C) of about 1 microliter (µL) to about 50 µL per square centimeter of the first exterior surface of the dressing composition. Thus, the amount of first citric acid solution applied may be about 1 µL, about 2 µL, about 3 µL, about 4 µL, about 5 µL, about 6 µL, about 7 µL, about 8 µL, about 9 µL, about 10 µL, about 11 µL, about 12 µL, about 13 µL, about 14 µL, about 15 µL, about 16 µL, about 17 µL, about 18 µL, about 19 µL, about 20 µL, about 21 µL, about 22 µL, about 23 µL, about 24 µL, about 25 µL, about 26 µL, about 27 µL, about 28 µL, about 29 µL, about 30 µL, about 32 µL, about 34 µL, about 36 µL, about 38 µL, about 40 µL, about 42 µL, about 44 µL, about 46 µL, about 48 µL, about 50 µL, or any range including and/or in between any two of these values. Such application may (but is not required to) occur at a temperature of about 20 °C to about 25 °C.

In any embodiment herein, the method may or may not further include applying a second citric acid solution (including a second solvent and about 50 mM to about 400 mM citric acid) to the second exterior surface of the dressing composition. The second citric acid solution may be applied at a volume (determined at 20 °C) of about 1 µL to about 50 µL per square centimeter of the second exterior surface of the dressing composition. Thus, the amount of second citric acid solution applied may be about 1 µL, about 2 µL, about 3 µL, about 4 µL, about 5 µL, about 6 µL, about 7 µL, about 8 µL, about 9 µL, about 10 µL, about 11 µL, about 12 µL, about 13 µL, about 14 µL, about 15 µL, about 16 µL, about 17 µL, about 18 µL, about 19 µL, about 20 µL, about 21 µL, about 22 µL, about 23 µL, about 24 µL, about 25 µL, about 26 µL, about 27 µL, about 28 µL, about 29 µL, about 30 µL, about 32 µL, about 34 µL, about 36 µL, about 38 µL, about 40 µL, about 42 µL, about 44 µL, about 46 µL, about 48 µL, about 50 µL, or any range including and/or in between any two of these values.

Applying the first citric acid solution includes spraying the first citric acid solution on a surface of a dressing composition (such as by use of an air brush and/or an ultrasonic sprayer). Applying the second citric acid solution (when present) may include spraying and/or coating of the second citric acid solution on a surface of a dressing composition (such as by use of an air brush and/or an ultrasonic sprayer).

The dressing composition may be according to any embodiment of a wound dressing composition of the present technology with the exception that the dressing composition does not include the first layer and does not include the second layer - thus, the method provides the first layer and (when present and as discussed herein) the second layer. Thus, the dressing composition includes about 30 wt.% to about 95 wt.% of one or more of collagen and chitosan, where the collagen may have a weight-average molecular weight of about 5,000 to about 100,000 and chitosan, and about 30 wt.% to about 70 wt.% ORC (where the ORC may have a weight-average molecular weight of about 50,000 to about 1,000,000), where a weight ratio of the one or more of collagen and chitosan to ORC is about 70:30 to about 30:70. It is to be understood that any component, value, and range/sub-range discussed in regard to any embodiment the wound dressing composition (absent, of course the first layer and when present the second layer) may be included in the dressing composition. Purely by way of example, U.S. Pat. Publ. 2001/0045177 provides procedures and protocols for producing dressing compositions.

As discussed above, the citric acidic solution includes a solvent and about 50 mM to about 400 mM citric acid. Thus, the concentration of citric acid in the citric acid solution may be about 50 mM, about 60 mM, about 70 mM, about 80 mM, about 90 mM, about 100 mM, about 110 mM, about 120 mM, about 130 mM, about 140 mM, about 150 mM, about 160 mM, about 170 mM, about 180 mM, about 190 mM, about 200 mM, about 220 mM, about 240 mM, about 260 mM, about 280 mM, about 300 mM, about 320 mM, about 340 mM, about 360 mM, about 380 mM, about 400 mM, or any range including and/or in between any two of the these values.

The first solvent and second solvent may each independently include one or more of water, ethanol, acetone, acetic acid, or a mixture of any two or more thereof. For example, in any embodiment herein, the first solvent and the second solvent may each independently include water. The amount of water (based on the total weight of the respective citric acid solution not considering the weight of citric acid) may independently be for the first and second citric acid solution about 10 wt.%, about 15 wt.%, about 20 wt.%, about 25 wt.%, about 30 wt.%, about 35 wt.%, about 40 wt.%, about 45 wt.%, about 50 wt.%, about 55 wt.%, about 60 wt.%, about 65 wt.%, about 70 wt.%, about 75 wt.%, about 80 wt.%, about 85 wt.%, about 90 wt.%, about 95 wt.%, about 100 wt.%, or any range including or in between any two of these values.

In any embodiment disclosed herein, the first solvent and second solvent may each independently include from about 10% to about 95% ethanol. For example, in any embodiment herein, the first solvent and the second solvent may each independently include ethanol present in an amount of about 10%, about 20%, about 30%, about 40%, about 50%, about 60%, about 70%, about 80%, about 90%, about 95%, or any range including and/or in between any two of the preceding values. In any embodiment disclosed herein, the first solvent and second solvent may each independently include about 70% ethanol.

In any embodiment disclosed herein, the first solvent and second solvent may each independently include about 0.01 M to about 0.1 M acetic acid. Thus, in any embodiment herein, the concentration of acetic acid may be about 0.01 M, about 0.02 M, about 0.03 M, about 0.04 M, about 0.05 M, about 0.06 M, about 0.07 M, about 0.08 M, about 0.09 M, about 0.1 M, or any range including and/or in between any two of the preceding values. For example, in any embodiment herein, the first solvent and second solvent may each independently include 0.05 M acetic acid.

The first citric acid solution and second citric acid solution may further each independently include a dye marker, such as E124, fluorescein isothiocyanate (FITC), 4',6-diamidino-2-phenylindole (DAPI), methylene blue, erythrosine B, ponceau S, alura red, SYBR green, alcian blue, brilliant blue G, calcein blue, cardio green, crystal violet, nile blue, india ink, brilliant blue, indigo carmine, sudan III, methyl green, oil red, pyronin Y, tattoo ink, purpurin, phloxine B, picric acid, carbon nanotubes, a fuchsin, resazurin, a trichrome, or a combination of any two or more thereof. In any embodiment herein, each dye marker may be independently included at a concentration of about 1 millimolar (mM) to about 100 mM. Thus, the concentration of each dye marker in each solution may independently be about 1 mM, about 2 mM, about 3 mM, about 4 mM, about 5 mM, about 6 mM, about 7 mM, about 8 mM, about 9 mM, about 10 mM, about 11 mM, about 12 mM, about 13 mM, about 14 mM, about 15 mM, about 16 mM, about 17 mM, about 18 mM, about 19 mM, about 20 mM, about 22 mM, about 24 mM, about 26 mM, about 28 mM, about 30 mM, about 32 mM, about 34 mM, about 36 mM, about 38 mM, about 40 mM, about 42 mM, about 44 mM, about 46 mM, about 48 mM, about 50 mM, about 55 mM, about 60 mM, about 65 mM, about 70 mM, about 75 mM, about 80 mM, about 85 mM, about 90 mM, about 95 mM, about 100 mM, or any range including and in between any two of these values.

The first citric acid solution and second citric acid solution may further each independently include an additive, such as acetic acid. In any embodiment herein, each additive may be independently included in the first citric acid solution at a concentration of about 0.01M to about 1 M. In any embodiment herein, each additive may be independently included in the second citric acid solution at a concentration of about 0.01M to about 1 M.

In any embodiment herein, the method may further include (subsequent to applying the first and (when present) second citric acid solution) a drying step to obtain the wound dressing composition. Such a drying step may be performed at a temperature of about 16 °C to about 40 °C. In any embodiment disclosed herein, the method may include drying the citric acid while the dressing composition is under tension. Suitable temperatures in any embodiment herein may include, but are not limited to, about 16 °C, about 17 °C, about 18 °C, about 19 °C, about 20 °C, about 21 °C, about 22 °C, about 23 °C, about 24 °C, about 25 °C, about 26 °C, about 27 °C, about 28 °C, about 29 °C, about 30 °C, about 31 °C, about 32 °C, about 33 °C, about 34 °C, about 35 °C, about 36 °C, about 37 °C, about 38 °C, about 39 °C, about 40 °C, or any range including and/or in between any two of the preceding values. For example, in any embodiment herein, the drying step may be performed at a temperature of about 20 °C to about 25 °C.

In a related aspect, the present technology provides a wound dressing composition prepared according to the methods described herein in any embodiment.

### Kits Including Wound Dressing Compositions of the Present Technology

In a further related aspect, the present disclosure provides kits that include a wound dressing composition of any embodiment described herein and instructions for use. The kit may optionally comprise components such as antiseptic wipes, ointment, adhesive tape, tweezers, scissors, *etc.* The instructions of any embodiment of a kit of the present technology may provide for a method of treating a wound in a subject.

The examples herein are provided to illustrate advantages of the present technology and to further assist a person of ordinary skill in the art with preparing or using the compositions and systems of the present technology. The examples should in no way be construed as limiting the scope of the present technology, as defined by the appended claims. The examples can include or incorporate any of the variations, aspects, or embodiments of the present technology described above. The variations, aspects, or embodiments described above may also further each include or incorporate the variations of any or all other variations, aspects or embodiments of the present technology.

### Negative-Pressure Therapy

The dressing of any embodiment described herein may be employed in therapy in which a tissue site is treated with reduced pressure. Treatment of tissue with reduced pressure may be commonly referred to as "negative-pressure therapy," but is also known by other names, including "negative-pressure wound therapy," "reduced-pressure therapy," "vacuum therapy," "vacuum-assisted closure," and "topical negative-pressure," for example. Negative-pressure therapy may provide a number of benefits, including migration of epithelial and subcutaneous tissues, improved blood flow, and/or micro-deformation of tissue at a wound site. Together, these benefits may increase development of granulation tissue and reduce healing times.

Generally, the system may be configured to provide negative-pressure to a tissue site in accordance with this specification. In any embodiment herein, the system may generally include a negative-pressure supply, and may include or be configured to be coupled to a distribution component. In general, a distribution component may refer to any complementary or ancillary component configured to be fluidly coupled to a negative-pressure supply in a fluid path between a negative-pressure supply and a tissue site.

In any embodiment herein, the dressing may be configured to distribute negative pressure. The dressing may comprise or be configured as a manifold. A "manifold" in this context generally includes any composition or structure providing a plurality of pathways configured to collect or distribute fluid across a tissue site under pressure. For example, a manifold may be configured to receive negative pressure from the negative-pressure source and to distribute negative pressure through multiple apertures (*e.g*., pores), which may have the effect of collecting fluid and drawing the fluid toward the negative-pressure source. Additionally or alternatively, the fluid path(s) may be reversed or a secondary fluid path may be provided to facilitate movement of fluid across a tissue site. Additionally or alternatively, the fluid pathways of a manifold may be interconnected to improve distribution or collection of fluids. Additionally or alternatively, a manifold may be a porous material having a plurality of interconnected cells or pores. For example, in any embodiment herein, open-cell foams such as reticulated foams may generally include pores, edges, and/or walls that may form interconnected fluid pathways (such as channels).

The fluid mechanics associated with using a negative-pressure source to reduce pressure in another component or location, such as within a sealed therapeutic environment, can be mathematically complex. However, the basic principles of fluid mechanics applicable to negative-pressure therapy are generally well-known to those skilled in the art. The process of reducing pressure may be described generally and illustratively herein as "delivering," "distributing," or "generating" negative pressure, for example.

In general, a fluid, such as wound fluid (for example, wound exudates and other fluids), flows toward lower pressure along a fluid path. Thus, the term "downstream" typically implies something in a fluid path relatively closer to a source of negative pressure or further away from a source of positive pressure. Conversely, the term "upstream" implies something relatively further away from a source of negative pressure or closer to a source of positive pressure. This orientation is generally presumed for purposes of describing various features and components herein. However, the fluid path may also be reversed in some applications (such as by substituting a positive-pressure source for a negative-pressure source) and this descriptive convention should not be construed as a limiting convention.

"Negative pressure" may generally refer to a pressure less than a local ambient pressure, such as the ambient pressure in a local environment external to a sealed therapeutic environment provided by the dressing. In many cases, the local ambient pressure may also be the atmospheric pressure proximate to or about a tissue site. Alternatively or additionally, the pressure may be less than a hydrostatic pressure associated with the tissue at the tissue site. While the amount and nature of negative pressure applied to a tissue site may vary according to therapeutic requirements, the pressure is generally a low vacuum, also commonly referred to as a rough vacuum, between -5 mm Hg (-667 Pa) and -500 mm Hg (-66.7 kPa), gauge pressure. Common therapeutic ranges are between -50 mm Hg (-6.7 kPa) and -300 mm Hg (-39.9 kPa), gauge pressure.

Additionally or alternatively, in any embodiment herein, a negative-pressure supply may be a reservoir of air at a negative pressure, or may be a manual or electrically-powered device that can reduce the pressure in a sealed volume, such as a vacuum pump, a suction pump, a wall suction port available at many healthcare facilities, or a micro-pump, for example. A negative-pressure supply may be housed within or used in conjunction with other components, such as sensors, processing units, alarm indicators, memory, databases, software, display devices, or user interfaces that further facilitate therapy. A negative-pressure source may be combined with a controller and other components into a therapy unit. A negative-pressure supply may also have one or more supply ports configured to facilitate coupling and de-coupling of the negative-pressure supply to one or more distribution components.

In any embodiment herein, components may be fluidly coupled to each other to provide a path for transferring fluids (*i.e.,* liquid and/or gas) between the components. For example, components may be fluidly coupled through a fluid conductor, such as a tube. As used herein, the term "fluid conductor" may include a tube, pipe, hose, conduit, or other structure with one or more lumina or open passages adapted to convey a fluid between two ends thereof. Typically, a fluid conductor may be an elongated, cylindrical structure with some flexibility, but the geometry and rigidity may vary. Additionally or alternatively, in any embodiment herein, the negative-pressure source may be operatively coupled to the dressing *via* a dressing interface.

### Methods of Treatment (not part of the claims)

The present technology also provides a therapy method, where the therapy method includes positioning a wound dressing composition of any embodiment herein of the present technology adjacent to the tissue site. For example, in any embodiment herein, the wound dressing may be positioned proximate to the wound. The wound dressing may be used with any of a variety of wounds, such as those occurring from trauma, surgery, or disease. For example, the wound dressing may be placed within, over, on, or otherwise proximate to the tissue site.

In any embodiment herein, the process may include employing the wound dressing in the context of a negative-pressure therapy, where the negative-pressure therapy may include positioning the wound dressing proximate to the tissue site (*e.g.,* a wound). For example, the various components of the dressing may be positioned with respect to the tissue site sequentially or, alternatively, may be positioned with respect to each other and then positioned with respect to the tissue site. The negative-pressure therapy may further comprise sealing the wound dressing to tissue surrounding the tissue site to form a sealed space. For example, the wound dressing may be positioned proximate to the tissue site and sealed to an attachment surface near the tissue site, for example, to undamaged epidermis peripheral to a tissue site.

The negative-pressure therapy method in any embodiment herein may further include fluidly coupling a negative-pressure source to the sealed space and operating the negative-pressure source to generate a negative pressure in the sealed space. For example, the negative-pressure source may be coupled to the dressing such that the negative-pressure source may be used to reduce the pressure in the sealed space. For example, negative pressure applied across the tissue site, for example, *via* the dressing may be effective to induce macrostrain and microstrain at the tissue site, as well as remove exudates and other fluids from the tissue site.

The present technology also provides a method of preventing formation of a biofilm or reducing biofilm at a tissue site, where the method includes applying a wound dressing composition of any embodiment herein of the present technology adjacent to the tissue site.

### EXAMPLES

### Example 1: Methods of Coating the Wound Dressing Compositions of the Present Technology

The following example illustrates how the compositions and methods of the present technology are surprisingly superior to wound dressing compositions provided by a common method of coating - dip Only the examples in which spray coating is used are according to the present invention, examples in which dip coating is used are comparative examples.

In particular, dressing compositions Promogran^{®} ("PN"; including a mixture of about 55 wt.% collagen and about 45 wt.% ORC) and Promogran Prisma^{®} ("PA"; including a mixture of about 55 wt.% collagen, about 44 wt.% ORC, and about 1 wt.% of a combination of silver in ORC where the combination includes about 25% w/w ionically bound silver) were utilized in the following studies, where the compositional details of each are provided in Table 1 below.

The citric acid solutions employed in the study were
- 100 mM citric acid in Barnstead water;
- 200 mM citric acid in Barnstead water;
- 100 mM citric acid in 70% ethanol (aq.);
- 200 mM citric acid in 70% ethanol (aq.);
- 100 mM citric acid in 0.05M acetic acid (aq.); and
- 200 mM citric acid in 0.05M acetic acid (aq.)
Controls were Barnstead water (18.2 MΩ), 70% ethanol (aq.), and 0.05M acetic acid (aq.).

For the dip coating studies, the dry weight of each dressing was first determined, followed by dip coating the dressing composition for 2 minutes in one of various citric acid solutions or controls, and subsequently removing the dip-coated dressing composition from the solution/control and air drying at room temperature (about 23 °C) for about 24 hours. Once the dip-coated dressing composition was dry, it was re-weighed. The results of the dip coating studies are illustrated in Table 2.

**Table 2: Dry weight of wound dressing compositions before and after dip coating with citric acid solutions.**

| **Water and Citric Acid** | | | | |
|---|---|---|---|---|
| | Sample | Citric Acid Concentration | Dry Weight | Weight After Dip Coating |
| 1 | PA | 100 mM | 0.204 g | 0.324 g |
| 2 | PN | 100 mM | 0.107 g | 0.198 g |
| 3 | PA | 200 mM | 0.208 g | 0.483 g |
| 4 | PN | 200 mM | 0.105 g | 0.322 g |
| 5 | PA | 0 mM | 0.210 g | 0.200 g |
| 6 | PN | 0 mM | 0.110g | 0.102g |

| **Water and Citric Acid in 70% EtOH** | | | | |
|---|---|---|---|---|
| | Sample | Citric Acid Concentration | Dry Weight | Weight After Dip Coating |
| 7 | PA | 100 mM | 0.205 g | 0.347 g |
| 8 | PN | 100 mM | 0.099 g | * |
| 9 | PA | 200 mM | 0.214 g | 0.512 g |
| 10 | PN | 200 mM | 0.104 g | 0.310 g |

| **Water and Citric Acid in 0.05M Acetic Acid** | | | | |
|---|---|---|---|---|
| | Sample | Citric Acid Concentration | Dry Weight | Weight After Dip Coating |
| 11 | PA | 100 mM | 0.203 g | 0.333 g |
| 12 | PN | 100 mM | 0.105 g | 0.192 g |
| 13 | PA | 200 mM | 0.214 g | 0.484 g |
| 14 | PN | 200 mM | 0.098 g | 0.231 g |

| | | | | |
|---|---|---|---|---|
| PA = PROMOGRAN PRISMA; PN = PROMOGRAN * Wound dressing could not be weighed after dip coating because it could not be separated from the drying tray. | | | | |

For the spray coating studies, the dry weight of each dressing was first determined, followed by spray coating the one surface of the dressing (about 28 cm² in surface area) with about 500 µL of one of the various citric acid solutions or controls, and subsequently allowing the spray-coated composition to air dry at room temperature (about 23 °C) for about 24 hours or oven dry at about 90 °C until dry (about 4-6 hours) under normal atmosphere. Once the spray-coated dressing composition was dry, it was re-weighed. The results of the spray coating studies are illustrated in Table 3 (for the air dried samples) and Table 4 (for the oven dried samples).

**Table 3: Dry weight of wound dressing compositions before and after spray coating with citric acid solutions.**

| **Room Temperature Samples** | | | | |
|---|---|---|---|---|
| **Water and Citric Acid** | | | | |
| | Sample | Citric Acid Concentration | Dry Weight | Weight After Spray Coating |
| 1 | PA | 100 mM | 0.190 g | 0.198 g |
| 2 | PN | 100 mM | 0.096 g | 0.106 g |
| 3 | PA | 200 mM | 0.186 g | 0.198 g |
| 4 | PN | 200 mM | 0.092 g | 0.108 g |
| 5 | PA | 0 mM | 0.193 g | 0.193 g |
| 6 | PN | 0 mM | 0.088 g | 0.089 g |

| **Water and Citric Acid in 70% EtOH** | | | | |
|---|---|---|---|---|
| | Sample | Citric Acid Concentration | Dry Weight | Weight After Spray Coating |
| 7 | PA | 100 mM | 0.190 g | 0.196 g |
| 8 | PN | 100 mM | 0.082 g | 0.089 g |
| 9 | PA | 200 mM | 0.183 g | 0.201 g |
| 10 | PN | 200 mM | 0.091 g | 0.107 g |
| 11 | PA | 0 mM | 0.195 g | 0.193 g |
| 12 | PN | 0 mM | 0.093 g | 0.094 g |

| **Water and Citric Acid in 0.05M Acetic Acid** | | | | |
|---|---|---|---|---|
| | Sample | Citric Acid Concentration | Dry Weight | Weight After Spray Coating |
| 13 | PA | 100 mM | 0.185 g | 0.198 g |
| 14 | PN | 100 mM | 0.094 g | 0.107 g |
| 15 | PA | 200 mM | 0.196 g | 0.222 g |
| 16 | PN | 200 mM | 0.096 g | 0.112 g |
| 17 | PA | 0 mM | 0.189 g | 0.189 g |
| 18 | PN | 0 mM | 0.093 g | 0.094 g |

| | | | | |
|---|---|---|---|---|
| PA = PROMOGRAN PRISMA; PN = PROMOGRAN | | | | |

**Table 4: Dry weight of wound dressing compositions before and after spray coating with citric acid solutions.**

| **Oven-Dried Samples (90 °C)** | | | | |
|---|---|---|---|---|
| **Water and Citric Acid** | | | | |
| | Sample | Citric Acid Concentration | Dry Weight | Weight After Spray Coating |
| 1 | PA | 0 mM | 0.206 g | 0.281 g |
| 2 | PN | 0 mM | 0.098 g | 0.094 g |

| **Water and Citric Acid in 70% EtOH** | | | | |
|---|---|---|---|---|
| | Sample | Citric Acid Concentration | Dry Weight | Weight After Spray Coating |
| 7 | PA | 100 mM | 0.218 g | 0.225 g |
| 8 | PN | 100 mM | 0.100 g | 0.102 g |
| 9 | PA | 200 mM | 0.213 g | 0.237 g |
| 10 | PN | 200 mM | 0.092 g | 0.115 g |
| 11 | PA | 0 mM | 0.237 g | 0.233 g |
| 12 | PN | 0 mM | 0.098 g | 0.087 g |

| **Water and Citric Acid in 0.05M Acetic Acid** | | | | |
|---|---|---|---|---|
| | Sample | Citric Acid Concentration | Dry Weight | Weight After Spray Coating |
| 13 | PA | 100 mM | 0.208 g | 0.213 g |
| 14 | PN | 100 mM | 0.083 g | 0.091 g |
| 15 | PA | 200 mM | 0.210 g | 0.214 g |
| 16 | PN | 200 mM | 0.096 g | 0.110 g |
| 17 | PA | 0 mM | 0.237 g | 0.409 g |
| 18 | PN | 0 mM | 0.108 g | 0.170 g |

| | | | | |
|---|---|---|---|---|
| PA = PROMOGRAN PRISMA; PN = PROMOGRAN | | | | |

The results demonstrate that the dip-coated wound dressings increased substantially in weight after dip coating. The dressings also shrunk about 50% or more of the original size of the dressing after drying for about 24 hours at room temperature. Without wishing to be bound by theory, it is believed that the dip coating process with a citric acid solution is altering the characteristics of the wound dressing compositions after drying. The results of the spray coating experiments indicate that the weight of the wound dressings did not change much after drying, whether at room temperature or in an oven. Surprisingly, the wound dressing did not shrink as much in size after spray coating compared to the dip coating method. The dressing samples allowed to dry at room temperature only demonstrated about 10-15% shrinkage compared to the original size of the dressing. Dressing samples that were allowed to dry in the oven at about 90 °C demonstrated about 20-50% shrinkage along with curling of the wound dressing. These data suggest that that the spray coating technique was superior to dip coating regardless of whether the dressing is air-dried or oven dried, but that spray coating coupled with air drying is especially advantageous for incorporation of a layer of citric acid into a dressing composition.

### Example 2_{:} Methods of Assessing Antimicrobial Activity for Wound Dressing Compositions of the Present Technology

The capability of wound dressing compositions disclosed herein in any embodiment (*e.g.*, wound dressings prepared according to Example 1) may be assessed using a zone of inhibition assay in which the dressings are exposed to petri dishes having a lawn of bacteria already present. Such bacteria is allowed to grow and the antimicrobial activity of the respective wound dressing compositions may be assessed. In particular, the effect on biofilms of such wound dressing compositions may be evaluated to determine biofilm populations after 24 hours of exposure with each respective wound dressing composition. Overall reduction in biofilm populations (*i.e.,* reduction in total viable count of the biofilm populations) following such exposure may be assessed and compared to untreated controls and comparative wound dressing compositions.

### EQUIVALENTS

It is intended that the specification be considered as exemplary only with the breadth and scope of the present technology indicated only by the appended claims, definitions therein and any equivalents thereof.

The embodiments, illustratively described herein may suitably be practiced in the absence of any element or elements, limitation or limitations, not specifically disclosed herein. Thus, for example, the terms "comprising," "including," "containing," etc. shall be read expansively and without limitation. Additionally, the terms and expressions employed herein have been used as terms of description and not of limitation, and there is no intention in the use of such terms and expressions of excluding any equivalents of the features shown and described or portions thereof. Additionally, the phrase "consisting essentially of" will be understood to include those elements specifically recited and those additional elements that do not materially affect the basic and novel characteristics of the claimed technology. The phrase "consisting of" excludes any element not specified.

In addition, where features or aspects of the disclosure are described in terms of Markush groups, those skilled in the art will recognize that the disclosure is also thereby described in terms of any individual member or subgroup of members of the Markush group. Each of the narrower species and subgeneric groupings falling within the generic disclosure also form part of the invention. This includes the generic description of the invention with a proviso or negative limitation removing any subject matter from the genus, regardless of whether or not the excised material is specifically recited herein.

As will be understood by one skilled in the art, for any and all purposes, particularly in terms of providing a written description, all ranges disclosed herein also encompass any and all possible subranges and combinations of subranges thereof. Any listed range can be easily recognized as sufficiently describing and enabling the same range being broken down into at least equal halves, thirds, quarters, fifths, tenths, etc. As a non-limiting example, each range discussed herein can be readily broken down into a lower third, middle third and upper third, etc. As will also be understood by one skilled in the art all language such as "up to," "at least," "down to," and the like, include the number recited and refer to ranges which can be subsequently broken down into subranges as discussed above. As will also be understood by one skilled in the art all language such as "greater than," "less than," and the like, refer to ranges which can be subsequently broken down into subranges as discussed above. Finally, as will be understood by one skilled in the art, a range includes each individual member.

Other embodiments are set forth in the following claims, along with the full scope of equivalents to which such claims are entitled.

## Claims

1. A wound dressing composition comprising
a first external surface and a second external surface;
about 30 wt.% to about 95 wt.% of one or more of collagen and chitosan;
about 30 wt.% to about 70 wt.% oxidized regenerated cellulose (ORC); and
a first layer disposed within the wound dressing composition adjacent to the first external
surface where the first layer further comprises citric acid, and wherein the first layer is a spray coated layer on the first external surface.

2. The wound dressing composition of claim 1, wherein the collagen has a weight-average molecular weight of about 5,000 to about 100,000.

3. The wound dressing composition of claim 1 or claim 2, wherein the ORC has a weight-average molecular weight of about 50,000 to about 1,000,000.

4. The wound dressing composition of any one of claims 1-3, wherein the wound dressing comprises a weight ratio of the one or more of collagen and chitosan to ORC is about 70:30 to about 30:70, optionally about 60:40 to about 40:60.

5. The wound dressing composition of any one of claims 1-4, wherein the first layer is at an average thickness of about 10 µm to about 3,500 µm.

6. The wound dressing composition of any one of claims 1-5, further comprising a second layer disposed within the wound dressing composition adjacent to the second external surface where the second layer further comprises citric acid, preferably wherein the second layer is a spray coated layer on the second external surface.

7. The wound dressing composition of claim 6, wherein the second layer is at an average thickness of about 150 µm to about 3,500 µm.

8. The wound dressing composition of any preceding claim, wherein the first layer and/or second layer comprises about 0.001 wt.% to about 20 wt.% citric acid based on total weight of the wound dressing composition.

9. The wound dressing composition of any one of claims 1-8, wherein the first layer and/or the second layer further comprises a dye marker, optionally wherein the dye marker of the first layer and/or second layer comprises one or more of E124, fluorescein isothiocyanate (FITC), 4',6-diamidino-2-phenylindole (DAPI), methylene blue, erythrosine B, ponceau S, alura red, SYBR green, alcian blue, brilliant blue G, calcein blue, cardio green, crystal violet, nile blue, india ink, brilliant blue, indigo carmine, sudan III, methyl green, oil red, pyronin Y, tattoo ink, purpurin, phloxine B, picric acid, carbon nanotubes, a fuchsin, resazurin, a trichrome, or a combination of any two or more thereof.

10. The wound dressing composition of any one of claims 1-9, wherein the wound dressing composition comprises collagen and the collagen is a mammalian collagen, optionally wherein the collagen comprises bovine collagen, human collagen, or a combination thereof.

11. The wound dressing composition of any one of claims 1-10, wherein the wound dressing compound comprises about 0.001 wt.% to about 5 wt.% ionic silver, optionally wherein the ionic silver comprises silver oxide, silver oxysalts, silver chromate, silver allantoinate, silver borate, silver glycerolate, silver nitrate, silver acetate, silver chloride, silver sulfate, silver lactate, silver bromide, silver iodide, silver carbonate, silver citrate, silver laurate, silver deoxycholate, silver salicylate, silver *p*-aminobenzoate, silver *p*-aminosalicylate, or a combination of any two or more thereof.

12. The wound dressing composition of any one of claims 1-11, wherein the wound dressing composition further comprises one or more of gelatin, fibronectin, hyaluronic acid, polysaccharides, and a combination of any two or more thereof.

13. A method of manufacturing a wound dressing composition from a dressing composition comprising a first exterior surface and a second exterior surface, the method comprising applying a first citric acid solution to the first exterior surface of the dressing composition by spray coating;
wherein
the dressing composition comprises
about 30 wt.% to about 95 wt.% of one or more of collagen and chitosan; about 30 wt.% to about 70 wt.% oxidized regenerated cellulose (ORC); and where a weight ratio of the one or more of collagen and chitosan to ORC is
about 70:30 to about 30:70; and
the first citric acidic solution comprises a first solvent and about 50 mM to about 400 mM citric acid.

14. The method of claim 13, wherein the method comprises applying about 1 µL to about 50 µL of the first citric acid solution per square centimeter of the first exterior surface.

15. The method of claim 13 or claim 14, wherein the first solvent comprises one or more of water, ethanol, and acetone.

16. The method of any one of claims 13-15, wherein the method further comprises applying a second citric acid solution to the second exterior surface, preferably by spray coating, where the second citric acid solution comprises a second solvent and about 50 mM to about 400 mM citric acid.

17. The method of claim 16, wherein the method comprises applying about 1 µL to about 50 µL of the second citric acid solution per square centimeter of the second exterior surface.

18. The method of claim 16 or claim 17, wherein the second solvent comprises one or more of water, ethanol, acetic acid, and acetone.

19. The method of claim 18, wherein the second solvent comprises 70% ethanol, 0.05 M acetic acid, or a mixture thereof.

20. The method of any one of claims 13-19, wherein the first and/or second citric acid solution comprises a dye marker.

21. The method of any one of claims 13-20, wherein the collagen of the dressing composition is a mammalian collagen.

22. The method of any one of claims 13-21, wherein the dressing composition comprises about 0.001 wt.% to about 5 wt.% ionic silver, optionally wherein the ionic silver comprises silver oxide, silver oxysalts, silver chromate, silver allantoinate, silver borate, silver glycerolate, silver nitrate, silver acetate, silver chloride, silver sulfate, silver lactate, silver bromide, silver iodide, silver carbonate, silver citrate, silver laurate, silver deoxycholate, silver salicylate, silver *p*-aminobenzoate, silver *p*-aminosalicylate, or a combination of any two or more thereof.

23. The method of any one of claims 13-22, wherein the dressing composition further comprises one or more of gelatin, fibronectin, hyaluronic acid, polysaccharides, and a combination of any two or more thereof.

24. The method of any one of claims 13-23, wherein the method further comprises drying the citric acid onto the dressing to obtain the wound dressing composition, optionally wherein the drying is carried out at temperatures from about 18°C to about 28°C.

25. A wound dressing composition prepared by a method according to any one of claims 13-24.

## Patentansprüche

1. Eine Wundverbandzusammensetzung, aufweisend
eine erste Außenoberfläche und eine zweite Außenoberfläche;
zu etwa 30 Gew.-% bis etwa 95 Gew.-% eines oder mehrere von Kollagen und Chitosan;
zu etwa 30 Gew.-% bis etwa 70 Gew.-% oxidierte regenerierte Cellulose (ORC); und
eine erste Schicht, die innerhalb der Wundverbandzusammensetzung angrenzend an die erste Außenoberfläche angeordnet ist, wobei die erste Schicht ferner Zitronensäure aufweist, und wobei die erste Schicht eine sprühbeschichtete Schicht auf der ersten Außenoberfläche ist.

2. Die Wundverbandzusammensetzung nach Anspruch 1, wobei das Kollagen ein gewichtsmittleres Molekulargewicht von etwa 5.000 bis etwa 100.000 aufweist.

3. Die Wundverbandzusammensetzung nach Anspruch 1 oder 2, wobei die ORC ein gewichtsmittleres Molekulargewicht von etwa 50.000 bis etwa 1.000.000 aufweist.

4. Die Wundverbandzusammensetzung nach einem der Ansprüche 1 bis 3, wobei der Wundverband ein Gewichtsverhältnis des einen oder der mehreren von Kollagen und Chitosan zu ORC aufweist, das etwa 70:30 bis etwa 30:70, optional etwa 60:40 bis etwa 40:60 beträgt.

5. Die Wundverbandzusammensetzung nach einem der Ansprüche 1 bis 4, wobei die erste Schicht eine durchschnittliche Dicke von etwa 10 µm bis etwa 3.500 µm aufweist.

6. Die Wundverbandzusammensetzung nach einem der Ansprüche 1 bis 5, ferner aufweisend eine zweite Schicht, die innerhalb der Wundverbandzusammensetzung angrenzend an die zweite Außenoberfläche angeordnet ist, wobei die zweite Schicht ferner Zitronensäure aufweist, vorzugsweise wobei die zweite Schicht eine sprühbeschichtete Schicht auf der zweiten Außenoberfläche ist.

7. Die Wundverbandzusammensetzung nach Anspruch 6, wobei die zweite Schicht eine durchschnittliche Dicke von etwa 150 µm bis etwa 3.500 µm aufweist.

8. Die Wundverbandzusammensetzung nach einem der vorhergehenden Ansprüche, wobei die erste Schicht und/oder die zweite Schicht zu etwa 0,001 Gew.-% bis etwa 20 Gew.-% Zitronensäure, bezogen auf das Gesamtgewicht der Wundverband, aufweist.

9. Die Wundverbandzusammensetzung nach einem der Ansprüche 1 bis 8, wobei die erste Schicht und/oder die zweite Schicht ferner eine Farbstoffmarkierung aufweist, optional wobei der Farbstoffmarker der ersten Schicht und/oder der zweiten Schicht eines oder mehrere von E124, Fluorescein-Isothiocyanat (FITC), 4',6-Diamidino-2-phenylindenol (DAPI), Methylenblau, Erythrosin B, Ponceau S, Alurarot, SYBR-Grün, Alcianblau, Brillantblau G, Calceinblau, Indocyaningrün, Kristallviolett, Nilblau, India-Tusche, Brillantblau, Indigocarmin, Sudan-III, Methylgrün, Ölrot, Pyronin-Y, Tattoo-Ink, Purpurin, Phloxin-B, Pikrinsäure, Kohlenstoffnanoröhrchen, Fuchsin, Resazurin, eine Trichrom-Färbung oder eine Kombination aus zwei beliebigen oder mehr davon aufweist.

10. Die Wundverbandzusammensetzung nach einem der Ansprüche 1 bis 9, wobei die Wundverbandzusammensetzung Kollagen aufweist und das Kollagen ein Säugetierkollagen ist, optional wobei das Kollagen Rinderkollagen, humanes Kollagen oder eine Kombination davon aufweist.

11. Die Wundverbandzusammensetzung nach einem der Ansprüche 1 bis 10, wobei die Wundverbandverbindung zu etwa 0,001 Gew.-% bis etwa 5 Gew.-% ionisches Silber aufweist, optional wobei das ionische Silber Silberoxid, Silberoxysalze, Silberchromat, Silberallantoin, Silberborat, Silberglycerolat, Silbernitrat, Silberacetat, Silberchlorid, Silbersulfat, Silberlactat, Silberbromid, Silberiodid, Silbercarbonat, Silbercitrat, Silberlaurat, Silberdesoxolat, Silbersalicylat, Silber-p-Aminobenzoat, Silber-p-Aminosalicylat oder eine Kombination aus beliebigen zwei oder mehr davon aufweist.

12. Die Wundverbandzusammensetzung nach einem der Ansprüche 1 bis 11, wobei die Wundverbandzusammensetzung ferner eines oder mehrere von Gelatine, Fibronectin, Hyaluronsäure, Polysacchariden und einer Kombination aus beliebigen zwei oder mehr davon aufweist.

13. Ein Verfahren zum Fertigen einer Wundverbandzusammensetzung aus einer Verbandzusammensetzung, aufweisend eine erste Außenoberfläche und eine zweite Außenoberfläche, das Verfahren aufweisend das Aufbringen einer ersten Zitronensäurelösung auf die erste Außenoberfläche der Verbandzusammensetzung durch Sprühbeschichten;
wobei
die Verbandzusammensetzung aufweist
zu etwa 30 Gew.-% bis etwa 95 Gew.-% eines oder mehrere von Kollagen und Chitosan;
zu etwa 30 Gew.-% bis etwa 70 Gew.-% oxidierte regenerierte Cellulose (ORC); und
wobei ein Gewichtsverhältnis des einen oder der mehreren von Kollagen und Chitosan zu ORC etwa 70:30 bis etwa 30:70 beträgt; und
die erste Zitronensäurelösung ein erstes Lösungsmittel und etwa 50 mM bis etwa 400 mM Zitronensäure aufweist.

14. Das Verfahren nach Anspruch 13, wobei das Verfahren das Aufbringen zu etwa 1 µl bis etwa 50 µl der ersten Zitronensäurelösung pro Quadratzentimeter der ersten Außenoberfläche aufweist.

15. Das Verfahren nach Anspruch 13 oder 14, wobei das erste Lösungsmittel eines oder mehrere von Wasser, Ethanol und Aceton aufweist.

16. Das Verfahren nach einem der Ansprüche 13 bis 15, wobei das Verfahren ferner das Aufbringen einer zweiten Zitronensäurelösung auf die zweite Außenoberfläche, vorzugsweise durch Sprühbeschichten, aufweist, wobei die zweite Zitronensäurelösung ein zweites Lösungsmittel und zu etwa 50 mM bis etwa 400 mM Zitronensäure aufweist.

17. Das Verfahren nach Anspruch 16, wobei das Verfahren das Aufbringen von etwa 1 µl bis etwa 50 µl der zweiten Zitronensäurelösung pro Quadratzentimeter der zweiten Außenoberfläche aufweist.

18. Das Verfahren nach Anspruch 16 oder 17, wobei das zweite Lösungsmittel eines oder mehrere von Wasser, Ethanol, Essigsäure und Aceton aufweist.

19. Das Verfahren nach Anspruch 18, wobei das zweite Lösungsmittel zu 70 % Ethanol, zu 0,05 M Essigsäure oder eine Mischung davon aufweist.

20. Das Verfahren nach einem der Ansprüche 13 bis 19, wobei die erste und/oder die zweite Zitronensäurelösung eine Farbstoffmarkierung aufweist.

21. Das Verfahren nach einem der Ansprüche 13 bis 20, wobei das Kollagen der Verbandzusammensetzung ein Säugetierkollagen ist.

22. Das Verfahren nach einem der Ansprüche 13 bis 21, wobei die Verbandzusammensetzung zu etwa 0,001 Gew.-% bis etwa 5 Gew.-% ionisches Silber aufweist, optional wobei das ionische Silber Silberoxid, Silberoxysalze, Silberchromat, Silberallantoin, Silberborat, Silberglycerolat, Silbernitrat, Silberacetat, Silberchlorid, Silbersulfat, Silberlactat, Silberbromid, Silberiodid, Silbercarbonat, Silbercitrat, Silberlaurat, Silberdesoxolat, Silbersalicylat, Silber-p-Aminobenzoat, Silber-p-Aminosalicylat oder eine Kombination aus beliebigen zwei oder mehr davon aufweist.

23. Das Verfahren nach einem der Ansprüche 13 bis 22, wobei die Verbandzusammensetzung ferner eines oder mehrere von Gelatine, Fibronectin, Hyaluronsäure, Polysacchariden und einer Kombination aus beliebigen zwei oder mehr davon aufweist.

24. Das Verfahren nach einem der Ansprüche 13 bis 23, wobei das Verfahren ferner ein Trocknen der Zitronensäure auf den Verband aufweist, um die Wundverbandzusammensetzung zu erhalten, optional wobei das Trocknen bei Temperaturen von etwa 18 °C bis etwa 28 °C durchgeführt wird.

25. Eine Wundverbandzusammensetzung, hergestellt durch ein Verfahren nach einem der Ansprüche 13 bis 24.

## Revendications

1. Composition de pansement pour plaie comprenant
une première surface externe et une seconde surface externe ;
environ 30 % en poids à environ 95 % en poids d'un ou plusieurs éléments parmi le collagène et le chitosane ;
environ 30 % en poids à environ 70 % en poids de cellulose régénérée oxydée (ORC) ; et
une première couche disposée à l'intérieur de la composition de pansement pour plaie adjacente à la première surface externe où la première couche comprend en outre de l'acide citrique, et dans laquelle la première couche est une couche appliquée par pulvérisation sur la première surface externe.

2. Composition de pansement pour plaie selon la revendication 1, dans laquelle le collagène a une masse moléculaire moyenne en poids d'environ 5 000 à environ 100 000.

3. Composition de pansement pour plaie selon la revendication 1 ou la revendication 2, dans laquelle l'ORC a une masse moléculaire moyenne en poids d'environ 50 000 à environ 1 000 000.

4. Composition de pansement pour plaie selon l'une quelconque des revendications 1 à 3, dans laquelle le pansement pour plaie comprend un rapport pondéral du ou des éléments parmi le collagène et le chitosane à l'ORC d'environ 70:30 à environ 30:70, facultativement d'environ 60:40 à environ 40:60.

5. Composition de pansement pour plaie selon l'une quelconque des revendications 1 à 4, dans laquelle la première couche a une épaisseur moyenne d'environ 10 µm à environ 3 500 µm.

6. Composition de pansement pour plaie selon l'une quelconque des revendications 1 à 5, comprenant en outre une seconde couche disposée dans la composition de pansement pour plaie adjacente à la seconde surface externe où la seconde couche comprend en outre de l'acide citrique, de préférence dans laquelle la seconde couche est une couche appliquée par pulvérisation sur la seconde surface externe.

7. Composition de pansement pour plaie selon la revendication 6, dans laquelle la seconde couche a une épaisseur moyenne d'environ 150 µm à environ 3 500 µm.

8. Composition de pansement pour plaie selon une quelconque revendication précédente, dans laquelle la première couche et/ou la seconde couche comprennent environ 0,001 % en poids à environ 20 % en poids d'acide citrique en fonction du poids total de la composition de pansement pour plaie.

9. Composition de pansement pour plaie selon l'une quelconque des revendications 1 à 8, dans laquelle la première couche et/ou la seconde couche comprennent en outre un colorant, facultativement dans laquelle le colorant de la première couche et/ou de la seconde couche comprend un ou plusieurs éléments parmi l'E124, l'isothiocyanate de fluorescéine (FITC), le 4',6-diamidino-2-phénylindole (DAPI), le bleu de méthylène, l'érythrosine B, le ponceau S, le rouge Allura, le vert SYBR, le bleu alcian, le bleu brillant G, le bleu calcéine, le vert cardio, le violet de cristal, le bleu du Nil, l'encre de Chine, le bleu brillant, le carmin d'indigo, le Soudan III, le vert de méthyle, le rouge pétrole, la pyronine Y, l'encre de tatouage, la purpurine, la phloxine B, l'acide picrique, les nanotubes de carbone, une fuchsine, la résazurine, un trichrome, ou une combinaison de deux quelconques de ceux-ci ou plus.

10. Composition de pansement pour plaie selon l'une quelconque des revendications 1 à 9, dans laquelle la composition de pansement pour plaie comprend du collagène et le collagène est un collagène de mammifère, facultativement dans laquelle le collagène comprend un collagène bovin, un collagène humain, ou une combinaison de ceux-ci.

11. Composition de pansement pour plaie selon l'une quelconque des revendications 1 à 10, dans laquelle la composition de pansement pour plaie comprend environ 0,001 % en poids à environ 5 % en poids d'argent ionique, facultativement dans laquelle l'argent ionique comprend de l'oxyde l'argent, des oxysels d'argent, du chromate d'argent, de l'allantoïnate d'argent, du borate d'argent, du glycérolate d'argent, du nitrate d'argent, de l'acétate d'argent, du chlorure d'argent, du sulfate d'argent, du lactate d'argent, du bromure d'argent, de l'iodure d'argent, du carbonate d'argent, du citrate d'argent, du laurate d'argent, du désoxycholate d'argent, du salicylate d'argent, du *p*-aminobenzoate d' argent, du *p*-aminosalicylate d'argent, ou une combinaison de deux quelconques de ceux-ci ou plus.

12. Composition de pansement pour plaie selon l'une quelconque des revendications 1 à 11, dans laquelle la composition de pansement pour plaie comprend en outre un ou plusieurs éléments parmi la gélatine, la fibronectine, l'acide hyaluronique, les polysaccharides et une combinaison de deux quelconques de ceux-ci ou plus.

13. Procédé de fabrication d'une composition de pansement pour plaie à partir d'une composition de pansement comprenant une première surface extérieure et une seconde surface extérieure, le procédé comprenant l'application d'une première solution d'acide citrique à la première surface extérieure de la composition de pansement par revêtement par pulvérisation ;
dans lequel
la composition de pansement comprend
environ 30 % en poids à environ 95 % en poids d'un ou plusieurs éléments parmi le collagène et le chitosane ;
environ 30 % en poids à environ 70 % en poids de cellulose régénérée oxydée (ORC) ; et
où un rapport pondéral du ou des éléments parmi le collagène et le chitosane à l'ORC est d'environ 70:30 à environ 30:70 ; et
la première solution d'acide citrique comprend un premier solvant et environ 50 mM à environ 400 mM d'acide citrique.

14. Procédé selon la revendication 13, dans lequel le procédé comprend l'application d'environ 1 µL à environ 50 µL de la première solution d'acide citrique par centimètre carré de la première surface extérieure.

15. Procédé selon la revendication 13 ou la revendication 14, dans lequel le premier solvant comprend un ou plusieurs éléments parmi l'eau, l'éthanol et l'acétone.

16. Procédé selon l'une quelconque des revendications 13 à 15, dans lequel le procédé comprend en outre l'application d'une seconde solution d'acide citrique à la seconde surface extérieure, de préférence par revêtement par pulvérisation, où la seconde solution d'acide citrique comprend un second solvant et environ 50 mM à environ 400 mM d'acide citrique.

17. Procédé selon la revendication 16, dans lequel le procédé comprend l'application d'environ 1 µL à environ 50 µL de la seconde solution d'acide citrique par centimètre carré de la seconde surface extérieure.

18. Procédé selon la revendication 16 ou la revendication 17, dans lequel le second solvant comprend un ou plusieurs éléments parmi l'eau, l'éthanol, l'acide acétique et l'acétone.

19. Procédé selon la revendication 18, dans lequel le second solvant comprend de l'éthanol à 70 %, de l'acide acétique à 0,05 M, ou un mélange de ceux-ci.

20. Procédé selon l'une quelconque des revendications 13 à 19, dans lequel la première et/ou la seconde solution d'acide citrique comprennent un colorant.

21. Procédé selon l'une quelconque des revendications 13 à 20, dans lequel le collagène de la composition de pansement est un collagène de mammifère.

22. Procédé selon l'une quelconque des revendications 13 à 21, dans lequel la composition de pansement comprend environ 0,001 % en poids à environ 5 % en poids d'argent ionique, facultativement dans lequel l'argent ionique comprend de l'oxyde d'argent, des oxysels d'argent, du chromate d'argent, de l'allantoïnate d'argent, du borate d'argent, du glycérolate d'argent, du nitrate d'argent, de l'acétate d'argent, du chlorure d'argent, du sulfate d'argent, du lactate d'argent, du bromure d'argent, de l'iodure d'argent, du carbonate d'argent, du citrate d'argent, du laurate d'argent, du désoxycholate d'argent, du salicylate d'argent, du *p*-aminobenzoate d'argent, du *p*-aminosalicylate d'argent, ou une combinaison de deux quelconques de ceux-ci ou plus.

23. Procédé selon l'une quelconque des revendications 13 à 22, dans lequel la composition de pansement comprend en outre un ou plusieurs éléments parmi la gélatine, la fibronectine, l'acide hyaluronique, les polysaccharides et une combinaison de deux quelconques de ceux-ci ou plus.

24. Procédé selon l'une quelconque des revendications 13 à 23, dans lequel le procédé comprend en outre le séchage de l'acide citrique sur le pansement pour obtenir la composition de pansement pour plaie, facultativement dans lequel le séchage est effectué à des températures allant d'environ 18 °C à environ 28 °C.

25. Composition de pansement pour plaie préparée par un procédé selon l'une quelconque des revendications 13 à 24.
